# EUROPEAN PATENT SPECIFICATION

(11) **EP 2 781 486 B1**
(45) Date of publication and mention of the grant of the patent: **02.08.2017**
(21) Application number: 12850506.2
(22) Date of filing: 14.11.2012
(51) Int. Cl.: C01G 9/02, A01N 59/16, A01P 3/00, A61K 8/27, A61Q 1/04, A61Q 1/08, A61Q 1/10, A61Q 1/12, A61Q 17/04, A61Q 19/00, C09C 1/04, A01N 25/26, A61Q 17/00, A61K 8/02, C08K 9/02, C08K 9/04, C08K 9/06

(54) **USE OF SURFACE-TREATED ZINC OXIDE POWDER AS ANTI-BACTERIAL AGENT**
VERWENDUNG VON OBERFLÄCHENBEHANDELTEM ZINKOXIDPULVER ALS ANTIBAKTERIKUM
UTILISATION DE POUDRE D'OXYDE DE ZINC À TRAITEMENT DE SURFACE COMME AGENT ANTIBACTÉRIEN

(30) Priority: 17.11.2011 JP 2011251935
(43) Date of publication of application: 24.09.2014
(73) Proprietor: Sakai Chemical Industry Co., Ltd., Sakai-shi, Osaka 590-8502 (JP)
(72) Inventor: HASHIMOTO, Mitsuo, Iwaki-shi Fukushima 971-8183 (JP); MAGARA, Koichiro, Iwaki-shi Fukushima 971-8183 (JP); NISHIDA, Kunitada, Sakai-shi Osaka 590-0985 (JP)
(74) Representative: Isarpatent
(86) International application number: PCT/JP2012/079471
(87) International publication number: WO 2013/073555

(56) References cited:
- EP-A1- 2 505 183
- WO-A1-00/42112
- WO-A1-2010/050139
- JP-A- H05 912
- JP-A- 2001 220 292
- JP-A- 2002 363 018
- JP-A- 2007 051 188
- JP-A- 2008 254 990
- JP-A- 2009 249 226
- US-A- 5 827 508

## Description

### TECHNICAL FIELD

The present disclosure relates to the use of surface treated zinc oxide powder as an antibacterial agent.

### BACKGROUND OF THE DISCLOSURE

Zinc oxide powder is used widely for various applications such as resins, films, and cosmetics. There is an antibacterial property as one of characteristics thereof. This antibacterial property of the zinc oxide powder gives the antibacterial property to products such as resins, films, and cosmetics.

Conventionally, zinc oxide powders to be used for various applications generally have an average particle diameter less than 1.1 µm, and such particles exerts suitable antibacterial property. However, zinc oxide microparticles having an average particle diameter of 1.1 µm or less are hard to disperse, and have a problem with the handleability due to low bulk specific gravity. In addition, there is a problem that the microparticles deteriorate the feelings in the cosmetic application. On the other hand, particles having the particle diameter of 1.1 µm or more is preferred because the particles may be used as a thermal conductive filler when used as an additive agent in a resin. Therefore, zinc oxide powders having the particle diameter of 1.1 µm or more have been developed and the use thereof for various applications has been examined, recently (Patent document 1 and 2).

However, zinc oxide powders, which are large particles having the average particle diameter of 1.1 µm or more, have no antibacterial effect, because the antibacterial effect is relative to the specific surface area of the zinc oxide powder. Therefore, the zinc oxide powders having the average particle diameter of 1.1 µm or more cannot be used for the applications requiring the antibacterial property.

It is publicly known that the zinc oxide powders having the average particle diameter of 1.1 µm or more, which are conventionally used widely, are surface treated (Patent documents 3 and 4). However, the surface treatment is performed mainly to prevent the elution of zinc ion, inhibit the chemical activity, and enhance the dispersion ability, not to improve the antibacterial performance.

US 5 827 508 relates to stable photoprotective compositions and describes the use of zinc oxide powders as photoprotective agents.

EP 2 505 183 A1 describes zinc oxide particles having a diameter of 0.1 to 10 µm, which have been surface treated with e.g. silane, in a cosmetic composition.

### PRIOR TECHNICAL DOCUMENTS

### PATENT DOCUMENTS

[Patent Document 1] WO 2011/043207
[Patent Document 2] Japanese Kokai Publication 2009-249226
[Patent Document 3] Japanese Kokai Publication Hei11-302015
[Patent Document 3] Japanese Kokai Publication 2007-16111

### SUMMARY OF INVENTION

### PROBLEMS TO BE SOLVED BY THE INVENTION

In view of the situations described above, it is an object of the present disclosure to provide zinc oxide powders having an average particle diameter of 1.1 µm or more and having an antibacterial property, an antibacterial agent comprising the same, and an antibacterial composition containing the same.

### MEANS FOR SOLVING OBJECT

The present disclosure relates to the use of surface treated zinc oxide powders as an antibacterial agent wherein the surface treated zinc oxide powders have an average particle diameter of 1.1 µm or more, and are surface treated by using at least one compound selected from the group consisting of silicon oxide, silicone oil, organic silicon compounds, and organic titanium compounds.

### EFFECTS OF THE INVENTION

The present disclosure relates to the use of surface treated zinc oxide powders as an antibacterial agent wherein the surface treated zinc oxide powders have an average particle diameter of 1.1 µm or more.

### DETAILED DESCRIPTION OF PREFERRED EMBODIMENTS

Herein described are surface treated zinc oxide powders which have an average particle diameter of 1.1 µm or more, and which are surface treated by using at least one compound selected from the group consisting of silicon oxide, silicone oil, organic silicon compounds, and organic titanium compounds. The antibacterial performance of zinc oxide powders having the average particle diameter of 1.1 µm or more considerably decreases and have substantially no bacterial property. However, as described herein zinc oxide having the antibacterial property can be obtained by surface treating the above-mentioned zinc oxide powder.

Silicon oxide, silicone oil, organic silicon compounds, organic titanium compounds, and silica and titanium oxide obtained by heat treating them are not compounds that have an antibacterial property, individually. Furthermore, it is not publicly known that the antibacterial property of zinc oxide is enhanced by coating with the above-mentioned compounds, and knowledge of this respect is completely unknown. As further described an unexpected effect can be obtained by forming the coating. The present invention relates to the use of the herein described surface treated zinc oxide powders as an antibacterial agent wherein the surface treated zinc oxide powders have an average particle diameter of 1.1 µm or more.

The present disclosure relates to the use of surface treated zinc oxide having an average particle diameter of 1.1 µm or more, and having a superior antibacterial performance. Zinc oxide powder having a superior antibacterial performance, despite large particle diameter, is not known until now.

The surface treated zinc oxide powders herein described have an average particle diameter of 1.1 µm or more. In the specification, the average particle diameter is measured by laser diffraction method. The effect of the present disclosure is especially remarkable for zinc oxide powders having an average particle diameter of 1 µm or more and that have not been surface treated, because particularly the antibacterial performance thereof deteriorates. The average particle diameter is more preferably 1.2 µm or more, still more preferably 1.3 µm or more. The upper limit of the average particle diameter is not particularly limited, but preferably 100 µm or less.

The surface treated zinc oxide powder as described herein is obtained by surface treating with at least one compound selected from the group consisting of silicon oxide, silicone oil, organic silicon compounds, and organic titanium compounds.

The silicon oxide is not particularly limited but may include silicon oxide obtained by neutralizing a water-soluble silicate.

The silicone oil is not particularly limited but may include triethoxycaprylylsilane (for example, AES-3083 manufactured by Shin-Etsu Chemical Co. , Ltd.), methyl hydrogen polysiloxane (for example, KF-99P manufactured by Shin-Etsu Chemical Co., Ltd., SH1107C manufactured by Dow Corning Toray Co. , Ltd.), dimethyl polysiloxane/methyl hydrogen polysiloxane copolymer (for example, KF-9901 manufactured by Shin-Etsu Chemical Co., Ltd.), triethoxysilylethyl polydimethylsiloxyethyl dimethicone (for example, KF-9908 manufactured by Shin-Etsu Chemical Co., Ltd.), triethoxysilylethyl polydimethylsiloxyethyl hexyl dimethicone (for example, KF-9909 manufactured by Shin-Etsu Chemical Co., Ltd.), acrylic silicone resin (for example, KP-574 manufactured by Shin-Etsu Chemical Co., Ltd.)

The organic silicon compound may include silane coupling agents, and alkoxysilanes. The silane coupling agent may include, for example, amino group-containing silan coupling agents such as γ-(2-aminoethyl)aminopropyl trimethoxysilane, γ-aminopropyl triethoxysilane, N-β(aminoethyl) γ-aminopropyl trimethoxysilane, and N-β(aminoethyl) γ-aminopropyl methyldimethoxysilane, glycidyl group-containing silane coupling agents such as γ-glycidoxypropyltrimethoxysilane, and γ-glycidoxypropylmethyldimethoxysilane, mercapto group-containing silane coupling agents such as γ-mercaptopropyl trimethoxysilane, vinyl group-containing silane coupling agents such as vinyltriethoxysilane, vinyltrimethoxysilane, and vinyltris(methoxyethoxy)silane, and (meth)acryloyl group-containing silane coupling agents such as γ- (meth)acryloyloxypropyltrimethoxysilane, γ-(meth)acryloyloxypropyltriethoxysilane, and γ-(meth)acryloyloxypropyldimethoxymethylsilane. The alkoxysilane may include methyltrimethoxysilane, dimethyldimethoxysilane, phenyltrimethoxysilane, diphenyldimethoxysilane, tetraethoxysilane, methyltriethoxysilane, dimethyldiethoxysilane, phenyltriethoxysilane, n-propyltrimethoxysilane, n-propyltriethoxysilane, hexyltrimethoxysilane, hexyltriethoxysilane, decyltrimethoxysilane, and trifluoropropyltrimethoxysilane.

The organic titanium compound may include di-i-propoxytitanium bis(methylacetoacetate), di-i-propoxytitanium bis(ethylacetoacetate), di-i-propoxytitanium bis(propylacetoacetate), di-i-propoxytitanium bis(butylacetoacetate), di-i-propoxytitanium bis(hexylacetoacetate), di-n-propoxytitanium bis(methylacetoacetate), di-n-propoxytitanium bis(ethylacetoacetate), di-n-propoxytitanium bis(propylacetoacetate), di-n-propoxytitanium bis(butylacetoacetate), di-n-propoxytitanium bis(hexylacetoacetate), di-n-butoxytitanium bis(methylacetoacetate), di-n-butoxytitanium bis(ethylacetoacetate), di-n-butoxytitanium bis(propylacetoacetate), di-n-butoxytitanium bis(butylacetoacetate), di-n-butoxytitanium bis(hexylacetoacetate), di-i-propoxytitanium bis(acetylacetonate), di-n-propoxytitanium bis(acetylacetonate), di-n-butoxytitanium bis(acetylacetonate), di-i-propoxytitanium bis(triethanolaminato), di-i-propoxytitanium bis(diethanolaminato), dibutoxytitanium bis(triethanolaminato), dibutoxytitanium bis(diethanolaminato), dioctyloxytitanium bis(octyleneglycolate), dihydroxytitanium bislactate, triisostearoyloxyisopropoxytitanium. Among them, titanium coupling agents are more preferred. Above all, di-i-propoxytitanium bis(acetylacetonate), di-i-propoxytitanium bis(triethanolaminato), and triisostearoyloxyisopropoxytitanium are especially preferred.

As for an untreated zinc oxide powder being a raw material of the surface treated zinc oxide powder as described herein, the shape and the production method thereof are not particularly limited as long as the average particle diameter thereof is 1.1 µm or more.

In addition, the surface treating method is not particularly limited, but any conventional method may be performed according to the respective surface treatments.

The treatment with a compound selected from silicon oxide, silicone oil, organic silicon compounds, and organic titanium compounds may be performed two or more times. In this case, different treatments may be performed successively.

In the surface treatment, a surface treatment layer consisting of the surface treatment agent is preferably formed in the range of 0.1 to 20 mass % relative to the total amount of the surface treated zinc oxide powder. When less than 0.1 mass %, it is not preferred because sufficient antibacterial performance may not be obtained. When exceeding 20 mass %, it is not preferred because the original performance of zinc oxide may be decreased.

The surface treated zinc oxide powder may be subjected to different surface treatment from the treatment with the compound selected from silicon oxide, silicone oil, organic silicon compounds, and organic titanium compounds. Zinc oxide powder subjected to the different surface treatment from the treatment with the compound selected from silicon oxide, silicone oil, organic silicon compounds, and organic titanium compounds may be surface treated with the compound selected from silicon oxide, silicone oil, organic silicon compounds, and organic titanium compounds.

The surface treated zinc oxide powder of the present disclosure has the performance as an antibacterial agent. Therefore, it may be added as an antibacterial agent into various compositions such as cosmetics, resin compositions, and coating compositions. The antibacterial performance can be provided when it is used for the applications utilizing other performances of zinc oxide than the antibacterial property, for example, it is used as a thermal conductive material, an ultraviolet shielding agent and so on.

The surface treated zinc oxide powder can be used suitably in cosmetics because it is superior in the antibacterial performance with an ultraviolet protecting performance and other performances of zinc oxide powder. The cosmetics may include skin cosmetics such as foundation, makeup base, eye shadow, cheek rouge, mascara, lipstick, sunscreen agent, milky lotion, cream, and lotion. The cosmetic can be in any form, for example, a form of an oil-based cosmetic, a water-based cosmetic, an O/W type cosmetic, or a W/O type cosmetic.

For the cosmetic any aqueous component or oily component that can be used in the field of cosmetics can be used in combination with the surface treated zinc oxide powder. The aqueous component and oily component described above are not particularly limited, and examples thereof may include those containing components such as oils, surfactants, moisturizers, higher alcohols, sequestrants, natural and synthetic polymers, water-soluble and oil-soluble polymers, UV blocking agents, various extracts, inorganic and organic pigments, inorganic and organic clay minerals and other powders, inorganic and organic pigments treated with metallic soap or silicone, coloring materials such as organic dyes, preservatives, antioxidants, dyes, thickeners, pH adjusters, perfumes, cooling-sensation agents, antiperspirants, disinfectants, and skin activators. Specifically, a desired cosmetic can be produced in the usual manner using any one or more of the components listed below. The amounts of these components incorporated are not particularly restricted as long as they do not interfere with the effects of the present disclosure.

The oil is not particularly limited, and examples thereof may include avocado oil, camellia oil, turtle oil, macadamia nut oil, corn oil, mink oil, olive oil, rapeseed oil, egg-yolk oil, sesame oil, persic oil, wheat germ oil, sasanqua oil, castor oil, linseed oil, safflower oil, cottonseed oil, perilla oil, soybean oil, arachis oil, tea seed oil, kaya oil, rice bran oil, Chinese tung oil, Japanese tung oil, jojoba oil, germ oil, triglycerol, glycerol trioctanoate, glycerol triisopalmitate, cacao butter, coconut oil, horse fat, hydrogenated coconut oil, palm oil, beef tallow, mutton tallow, hydrogenated beef tallow, palm kernel oil, lard, beef bone fat, Japan wax kernel oil, hydrogenated oil, neatsfoot oil, Japan wax, hydrogenated castor oil, beeswax, candelilla wax, cotton wax, carnauba wax, bayberry wax, insect wax, spermaceti wax, montan wax, bran wax, lanolin, kapok wax, lanolin acetate, liquid lanolin, sugarcane wax, isopropyl lanolate, hexyl laurate, reduced lanolin, jojoba wax, hard lanolin, shellac wax, POE lanolin alcohol ether, POE lanolin alcohol acetate, POE cholesterol ether, polyethylene glycol lanolate, POE hydrogenated lanolin alcohol ether, liquid paraffin, ozokerite, pristane, paraffin, ceresin, squalene, Vaseline, and microcrystalline wax.

The lipophilic nonionic surfactant is not particularly limited, and examples thereof may include sorbitan fatty acid esters such as sorbitan monooleate, sorbitan monoisostearate, sorbitan monolaurate, sorbitan monopalmitate, sorbitan monostearate, sorbitan sesquioleate, sorbitan trioleate, diglycerol sorbitan penta-2-ethylhexylate, and diglycerol sorbitan tetra-2-ethylhexylate; glycerin polyglycerin fatty acids such as glycerol mono-cottonseed oil fatty acid, glycerol monoerucate, glycerol sesquioleate, glycerol monostearate, α,α'-glycerol oleate pyroglutamate, and glycerol monostearate malate; propylene glycol fatty acid esters such as propylene glycol monostearate; hydrogenated castor oil derivatives; and glycerol alkyl ethers.

The hydrophilic nonionic surfactant is not particularly limited, and examples thereof may include POE sorbitan fatty acid esters such as POE sorbitan monooleate, POE sorbitan monostearate and POE sorbitan tetraoleate; POE sorbitol fatty acid esters such as POE sorbitol monolaurate, POE sorbitol monooleate, POE sorbitol pentaoleate and POE sorbitol monostearate; POE glycerin fatty acid esters such as POE glycerin monostearate, POE glycerin monoisostearate and POE glycerin triisostearate; POE fatty acid esters such as POE monooleate, POE distearate, POE monodioleate and ethylene glycol distearate; POE alkyl ethers such as POE lauryl ether, POE oleyl ether, POE stearyl ether, POE behenyl ether, POE 2-octyldodecyl ether and POE cholestanol ether; POE alkyl phenyl ethers such as POE octyl phenyl ether, POE nonyl phenyl ether and POE dinonyl phenyl ether; Pluaronic types such as Pluronic; POE/POP alkyl ethers such as POE/POP cetyl ether, POE/POP 2-decyltetradecyl ether, POE/POP monobutyl ether, POE/POP hydrogenated lanolin and POE/POP glycerin ether; tetra-POE/tetra-POP ethylenediamine condensation products such as Tetronic; POE castor oil hydrogenated castor oil derivatives such as POE castor oil, POE hydrogenated castor oil, POE hydrogenated castor oil monoisostearate, POE hydrogenated castor oil triisostearate, POE hydrogenated castor oil monopyroglutamic acid monoisostearic acid diester and POE hydrogenated castor oil maleic acid; POE beeswax/lanolin derivatives such as POE sorbitol beeswax; alkanolamides such as coconut oil fatty acid diethanolamide, lauric acid monoethanolamide and fatty acid isopropanol amide; POE propylene glycol fatty acid esters; POE alkylamines; POE fatty acid amides; sucrose fatty acid esters; POE nonylphenyl formaldehyde condensation products; alkyl ethoxy dimethylamine oxides; and trioleyl phosphoric acid.

Examples of other surfactants include anionic surfactants such as fatty acid soaps, higher-alkyl sulfuric ester salts, POE triethanolamine lauryl sulfate, and alkyl ether sulfuric ester salts; cationic surfactants such as alkyl trimethylammonium salts, alkyl pyridinium salts, alkyl quaternary ammonium salts, alkyl dimethylbenzyl ammonium salts, POE alkylamines, alkylamine salts, and polyamine fatty acid derivatives; and amphoteric surfactants such as imidazoline amphoteric surfactants and betaine surfactants. They may be incorporated within the bounds of not causing any problems with stability and skin irritation.

The moisturizer is not particularly limited, and examples thereof may include xylitol, sorbitol, maltitol, chondroitin sulfate, hyaluronic acid, mucoitinsulfuric acid, caronic acid, atelocollagen, cholesteryl-12-hydroxystearate, sodium lactate, bile salts, dl-pyrrolidone carboxylate, short-chain soluble collagens, diglycerol (EO) PO adducts, Rosa roxburghii extract, yarrow extract, and melilot extract.

The higher alcohol is not particularly limited, and examples thereof may include linear alcohols such as lauryl alcohol, cetyl alcohol, stearyl alcohol, behenyl alcohol, myristyl alcohol, oleyl alcohol, and cetostearyl alcohol; and branched alcohols such as monostearyl glycerol ether (batyl alcohol), 2-decyltetradecynol, lanolin alcohol, cholesterol, phytosterol, hexyldodecanol, isostearyl alcohol, and octyldodecanol.

The sequestrant is not particularly limited, and examples thereof may include 1-hydroxyethane-1,1-diphosphonic acid, 1-hydroxyethane-1,1-diphosphonic acid tetrasodium salt, sodium citrate, sodium polyphosphate, sodium metaphosphate, gluconic acid, phosphoric acid, citric acid, ascorbic acid, succinic acid, and edetic acid.

The natural water-soluble polymer is not particularly limited, and examples thereof may include plant-derived polymers such as gum arabic, tragacanth gum, galactan, guar gum, carob gum, karaya gum, carrageenan, pectin, agar, quince seed (quince), algal colloid (algal extract), starch (rice, corn, potato, wheat), and glycyrrhizinic acid; microorganism-derived polymers such as xanthan gum, dextran, succinoglucan, and pullulan; and animal-derived polymers such as collagen, casein, albumin, and gelatin.

The semisynthetic water-soluble polymer is not particularly limited, and examples thereof may include starch polymers such as carboxymethyl starch and methyl hydroxypropyl starch; cellulose polymers such as methyl cellulose, nitro cellulose, ethyl cellulose, methyl hydroxypropyl cellulose, hydroxyethyl cellulose, cellulose sodium sulfate, hydroxypropyl cellulose, sodium carboxymethylcellulose (CMC), crystalline cellulose, and cellulose powder; and alginate polymers such as sodium alginate and propylene glycol alginate.

The synthetic water-soluble polymer is not particularly limited, and examples thereof may include vinyl polymers such as polyvinyl alcohol, polyvinyl methyl ether, and polyvinyl pyrrolidone; polyoxyethylene polymers such as polyethylene glycol 20,000, polyethylene glycol 40,000, and polyethylene glycol 60,000; copolymers such as polyoxyethylene-polyoxypropylene copolymers; acrylic polymers such as sodium polyacrylate, polyethylacrylate, and polyacrylamide; polyethyleneimine; and cationic polymers.

The inorganic water-soluble polymer is not particularly limited, and examples thereof may include bentonite, magnesium aluminum silicate (Veegum), laponite, hectorite, and silicic anhydride.

The ultraviolet blocking agent is not particularly limited, and examples thereof may include benzoic acid-based ultraviolet blocking agents such as paraaminobenzoic acid (hereinafter, abbreviated as PABA), PABA monoglycerin ester, N,N-dipropoxy PABA ethyl ester, N,N-diethoxy PABA ethyl ester, N,N-dimethyl PABA ethyl ester and N,N-dimethyl PABA butyl ester; anthranilic acid-based ultraviolet blocking agents such as homomenthyl-N-acetyl anthranilate; salicylic acid-based ultraviolet blocking agents such as amyl salicylate, menthyl salicylate, homomenthyl salicylate, octyl salicylate, phenyl salicylate, benzyl salicylate and p-isopropanol phenyl salicylate; cinnamic acid-based ultraviolet blocking agents such as octyl cinnamate, ethyl-4-isopropyl cinnamate, methyl-2,5-diisopropyl cinnamate, ethyl-2,4-diisopropyl cinnamate, methyl-2,4-diisopropyl cinnamate, propyl-p-methoxy cinnamate, isopropyl-p-methoxy cinnamate, isoamyl-p-methoxy cinnamate, 2-ethoxyethyl-p-methoxy cinnamate, cyclohexyl-p-methoxy cinnamate, ethyl-α-cyano-β-phenyl cinnamate, 2-ethylhexyl-α-cyano-β-phenyl cinnamate and glycerylmono-2-ethylhexanoyl-diparamethoxy cinnamate; benzophenone-based ultraviolet blocking agents such as 2,4-dihydroxybenzophenone, 2,2'-dihydroxy-4-methoxybenzophenone, 2,2'-dihydroxy-4,4'-dimethoxybenzophenone, 2,2',4,4'-tetrahydroxybenzophenone, 2-hydroxy-4-methoxybenzophenone, 2-hydroxy-4-methoxy-4'-methylbenzophenone, 2-hydroxy-4-methoxybenzophenone-5-sulfonate, 4-phenylbenzophenone, 2-ethylhexyl-4'-phenyl-benzophenone-2-carboxylate, 2-hydroxy-4-n-octoxybenzophenone and 4-hydroxy-3-carboxybenzophenone; 3-(4'-methylbenzylidene)-d,l-camphor, 3-benzylidene-d,l-camphor, urocanic acid, urocanic acid ethyl ester, 2-phenyl-5-methylbenzoxazole, 2,2'-hydroxy-5-methylphenyl benzotriazole, 2-(2'-hydroxy-5'-t-octylphenyl)benzotriazole, 2-(2'-hydroxy-5'-methylphenyl) benzotriazole, dibenzalazine, dianisoylmethane, 4-methoxy-4'-t-butyldibenzoylmethane and 5-(3,3-dimethyl-2-norbornylidene)-3-pentane-2-one.

Other chemical components are not particularly limited, and examples thereof may include vitamins such as vitamin A oil, retinol, retinol palmitate, inositol, pyridoxine hydrochloride, benzyl nicotinate, nicotinamide, DL-α-tocopherol nicotinate, magnesium ascorbyl phosphate, 2-O-α-D-glucopyranosyl-L-ascorbic acid, vitamin D2 (ergocalciferol), DL-α-tocopherol, DL-α-tocopherol acetate, pantothenic acid, and biotin; hormones such as estradiol and ethynyl estradiol; amino acids such as arginine, aspartic acid, cystine, cysteine, methionine, serine, leucine, and tryptophan; anti-inflammatory agents such as allantoin and azulene; whitening agents such as arbutin; astringents such as tannic acid; refrigerants such as L-menthol and camphor, sulfur, lysozyme chloride, and pyridoxine chloride.

Various kinds of extracts are not particularly limited, and examples thereof may include Houttuynia cordata extract, Phellodendron bark extract, melilot extract, dead nettle extract, licorice extract, peony root extract, soapwort extract, luffa extract, cinchona extract, strawberry geranium extract, sophora root extract, nuphar extract, fennel extract, primrose extract, rose extract, rehmannia root extract, lemon extract, lithospermum root extract, aloe extract, calamus root extract, eucalyptus extract, field horsetail extract, sage extract, thyme extract, tea extract, seaweed extract, cucumber extract, clove extract, bramble extract, lemon balm extract, carrot extract, horse chestnut extract, peach extract, peach leaf extract, mulberry extract, knapweed extract, hamamelis extract, placenta extract, thymic extract, silk extract, and licorice extract.

Examples of the various kinds of powders may include bright coloring pigments such as red oxide, yellow iron oxide, black iron oxide, mica titanium, iron oxide-coated mica titanium and titanium oxide-coated glass flakes, inorganic powders such as those of mica, talc, kaolin, sericite, titanium dioxide and silica, and organic powders such as polyethylene powder, nylon powder, crosslinked polystyrene, cellulose powder and silicone powder. Preferably, part or all of the powder component is subjected to a hydrophobization treatment by well-known method with a substance such as a silicone, a fluorine compound, a metallic soap, an oily agent or an acyl glutamic acid salt for improvement of sensory characteristics and improvement of makeup retainability. Other zinc oxide powders may be mixed and used.

### EXAMPLES

Hereinafter, the present disclosure will be explained with reference to examples. However, the present disclosure is not limited to these examples.

### Preparation of test piece for antibacterial test

Polyethylene resin (Sumikathene F-412-1) 100 g and sample 20 g were kneaded for 5 minutes using a twin roll heated to 115°C. At this time, a clearance of the twin roll was set at 0.2 mm. After kneading, the obtained composition was pressed at 100°C for 10 minutes by using a steam press, and cooled to 60°C to obtain a film-like composition. The composition was pressed so that the film thickness was 1 mm, and after pressing, the film-like composition was cut into a square shape having a side of 100 mm to make a test piece for an antibacterial test.

### Example 1

Large particle zinc oxide LPZINC-2 (manufactured by Sakai Chemical Industry Co., Ltd., average particle diameter 2 µm) 100 g and silicone oil (KF-9901 manufactured by Shin-Etsu Chemical Co., Ltd.) 1 g were put into a 300 ml mayonnaise bottle and shaked for 20 minutes by using a paint conditioner to coat uniformly. Then, heat treatment was conducted at 105°C for 12 hours to obtain surface treated zinc oxide particles. A test piece for an antibacterial test was prepared from the obtained surface treated zinc oxide particles by the same procedure described above. The results of the antibacterial test were shown in table 1.

### Example 2

Large particle zinc oxide LPZINC-2 (manufactured by Sakai Chemical Industry Co., Ltd., average particle diameter 2 µm) 100 g and decyltrimethoxysilane (KBM-3103C manufactured by Shin-Etsu Chemical Co., Ltd.) 1 g were put into a 300 ml mayonnaise bottle and shaked for 20 minutes by using a paint conditioner to coat uniformly. Then, heat treatment was conducted at 105°C for 12 hours to obtain surface treated zinc oxide particles. A test piece for an antibacterial test was prepared from the obtained surface treated zinc oxide particles by the same procedure described above. The results of the antibacterial test were shown in table 1.

### Example 3

Large particle zinc oxide LPZINC-2 (manufactured by Sakai Chemical Industry Co., Ltd., average particle diameter 2 µm) 100 g and titanium coupling agent (KR-TTS manufactured by Ajinomoto Fine-Techno Co., Inc.) 1 g were put into a 300 ml mayonnaise bottle and shaked for 20 minutes by using a paint conditioner to coat uniformly. Then, heat treatment was conducted at 105°C for 12 hours to obtain surface treated zinc oxide particles. A test piece for an antibacterial test was prepared from the obtained surface treated zinc oxide particles by the same procedure described above. The results of the antibacterial test were shown in table 1.

### Example 4

Large particle zinc oxide LPZINC-11 (manufactured by Sakai Chemical Industry Co., Ltd., average particle diameter 11 µm) was dispersed in 1L pure water, and sodium silicate aqueous solution (with SiO₂ of 29%) 3.5 g was added. Then, 0.1% sulfuric acid was added until pH became 7.0, and SiO₂ was precipitated on the particle surface. Thereafter, surface treated zinc oxide particles were obtained by filtering and drying. A test piece for an antibacterial test was prepared from the obtained surface treated zinc oxide particles by the same procedure described above. The results of the antibacterial test were shown in table 1.

### Example 5

Large particle zinc oxide LPZINC-11 (manufactured by Sakai Chemical Industry Co., Ltd., average particle diameter 11 µm) 100 g and silicone oil (KF-9901 manufactured by Shin-Etsu Chemical Co., Ltd.) 1 g were put into a 300 ml mayonnaise bottle and shaked for 20 minutes by using a paint conditioner to coat uniformly. Then, heat treatment was conducted at 105°C for 12 hours to obtain surface treated zinc oxide particles. A test piece for an antibacterial test was prepared from the obtained surface treated zinc oxide particles by the same procedure described above. The results of the antibacterial test were shown in table 1.

### Comparative example 1

A test piece for an antibacterial test was prepared from large particle zinc oxide LPZINC-2 (manufactured by Sakai Chemical Industry Co., Ltd., average particle diameter 2 µm) that is not surface treated by the same procedure described above. The results of the antibacterial test were shown in table 1.

### Comparative example 2

A test piece for an antibacterial test was prepared from large particle zinc oxide LPZINC-11 (manufactured by Sakai Chemical Industry Co., Ltd., average particle diameter 11 µm) that is not surface treated by the same procedure described above. The results of the antibacterial test were shown in table 1.

### Comparative example 3

A test piece for an antibacterial test was prepared from spherical SiO₂ (manufactured by Sakai Chemical Industry Co., Ltd., average particle diameter 0.7 µm) that is not surface treated by the same procedure described above. The results of the antibacterial test were shown in table 1.

### Comparative example 4

Spherical SiO₂ (manufactured by Sakai Chemical Industry Co., Ltd., average particle diameter 0.7 µm) 100 g and silicone oil (KF-9901 manufactured by Shin-Etsu Chemical Co., Ltd.) 1 g were put into a 300 ml mayonnaise bottle and shaked for 20 minutes by using a paint conditioner to coat uniformly. Then, heat treatment was conducted at 105°C for 12 hours to obtain surface treated silica particles. A test piece for an antibacterial test was prepared from the obtained surface treated silica particles by the same procedure described above. The results of the antibacterial test were shown in table 1.

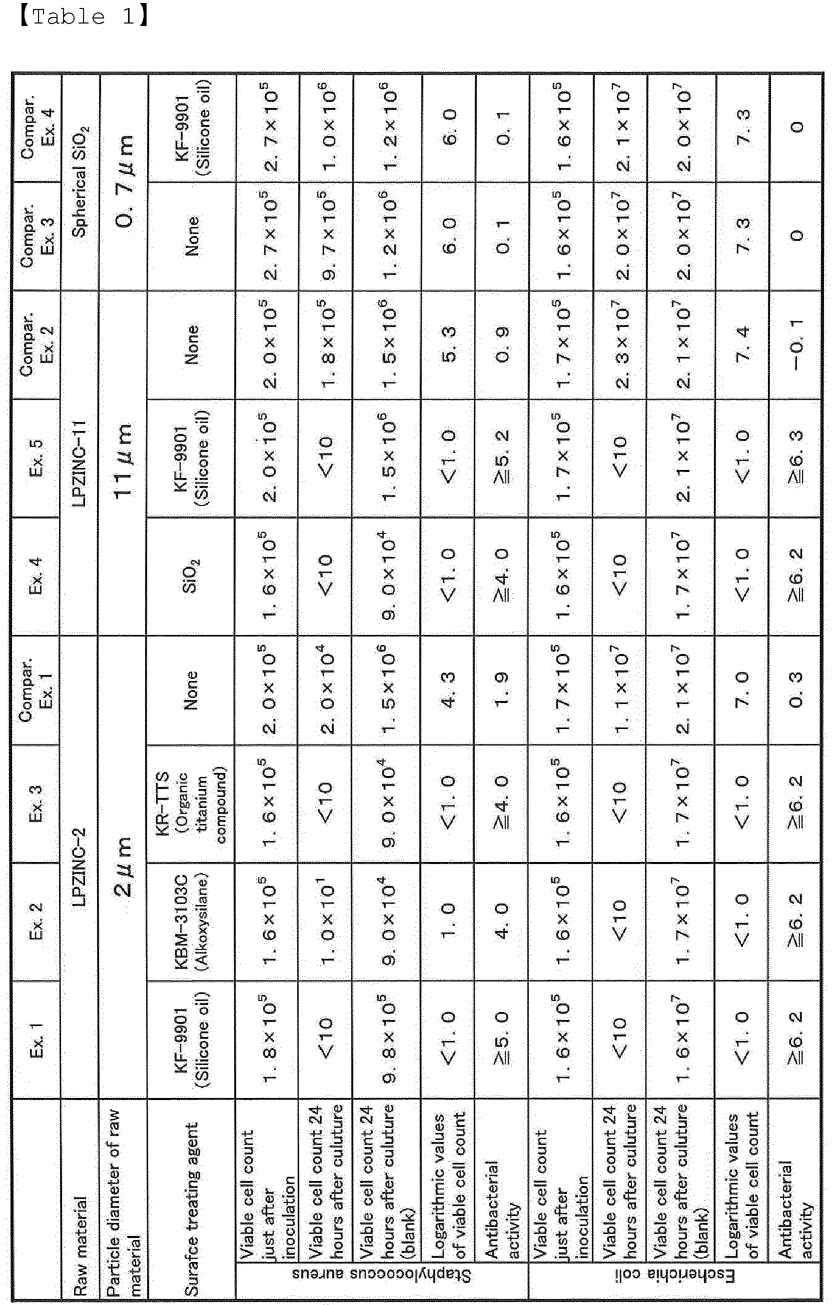

From the results of table 1, zinc oxide powders having particle diameter of 1.1 µm or more not subjected to a surface treatment have no antibacterial performance. On the other hand, it is clear that all of the surface treated zinc oxide powder of the present disclosure have the antibacterial performance. Furthermore, it is clear that, if silica particles are subjected to the same surface treatment, the antibacterial performance cannot be given. From these results, it is clear that the antibacterial performance is produced by the interaction occurred by surface coating the zinc oxide powder not only the silica coating.

### INDUSTRIAL APPLICABILITY

The surface treated zinc oxide powder may be used as an antibacterial agent for cosmetics.

## Claims

1. Use of surface treated zinc oxide powders as an antibacterial agent wherein the surface treated zinc oxide powders have an average particle diameter of 1.1 µm or more, and
are surface treated by using at least one compound selected from the group consisting of silicon oxide, silicone oil, organic silicon compounds, and organic titanium compounds.

## Patentansprüche

1. Verwendung von oberflächenbehandelten Zinkoxidpulvern als antibakterielle Mittel, wobei die oberflächenbehandelten Zinkoxidpulver einen durchschnittlichen Teilchendurchmesser von 1,1 µm oder mehr haben, und unter Verwendung von wenigstens einer Verbindung ausgewählt aus der Gruppe bestehend aus Siliciumoxid, Silikonöl, organischen Siliciumverbindungen, und organischen Titanverbindungen oberflächenbehandelt sind.

## Revendications

1. Utilisation de poudres d'oxyde de zinc à traitement de surface en tant qu'agent antibactérien, dans laquelle les poudres d'oxyde zinc à traitement de surface ont un diamètre moyen de particules de 1,1 µm ou plus, et
ont reçu un traitement de surface à l'aide d'au moins un composé choisi dans le groupe constitué d'oxyde de silicium, d'huile de silicone, de composés d'organo-silicium et de composés d'organo-titane.
